(19) Europäisches Patentamt
European Patent Office
Office européen des brevets

(11) Publication number: **0 078 518**
**B1**

(12) **EUROPEAN PATENT SPECIFICATION**

(45) Date of publication of patent specification: **30.01.85**

(51) Int. Cl.$^4$: **C 07 C 93/24,** A 61 K 31/215

(21) Application number: **82109995.9**

(22) Date of filing: **28.10.82**

(54) **A new p-chlorophenoxyacetic acid derivative, its method of preparation and pharmaceutical composition.**

(30) Priority: **29.10.81 ES 506669**

(43) Date of publication of application:
**11.05.83 Bulletin 83/19**

(45) Publication of the grant of the patent:
**30.01.85 Bulletin 85/05**

(84) Designated Contracting States:
**AT BE CH DE FR GB IT LI NL SE**

(56) References cited:
**BE-A- 669 202**

(73) Proprietor: **SOCIEDAD ESPANOLA DE ESPECIALIDADES FARMACO-TERAPEUTICAS, S.A.**
**173, Avda. San Antonio Ma Claret**
**Barcelona-26 (ES)**

(72) Inventor: **Andreoli, Romeo R., Dipl.-Chem.**
**6, calle Roca i Batlle**
**Barcelona (ES)**
Inventor: **Cirera, Xavier D.**
**271, Avenida Republica Argentina**
**Barcelona (ES)**

(74) Representative: **Berg, Wilhelm, Dr. et al**
**Patentanwälte Dr. Berg Dipl.-Ing. Stapf Dipl.-Ing. Schwabe Dr. Dr. Sandmair Postfach 86 02 45**
**Stuntzstrasse 16**
**D-8000 München 86 (DE)**

**EP 0 078 518 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This present invention refers to a new compound derived from p-chlorophenoxyacetic acid, the p-chlorophenoxyacetate of 1-N-(2-hydroxyethyl-1)-aminoadamantane of structural formula I

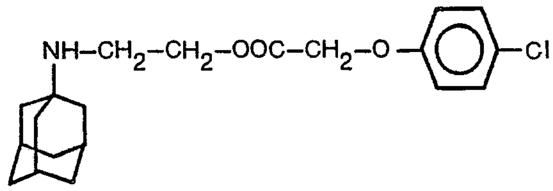

I

The invention also refers to the salts thereof, which are of pharmacological interest, to its method of preparation, to the pharmaceutical compositions containing it and to its therapeutic applications.

The compound I and its salt are new agents which are active on the central nervous system, being of use in the treatment of various pathological conditions of the system.

Method of Preparation

To prepare the p-chlorophenoxyacetate of N-(adamantyl-1)-2-aminoethanol the esterification reaction is carried out between the acylic compound of the following structural formula II

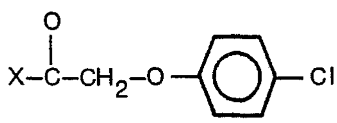

II

where X can be an OH group or a halogen atom (Cl or Br), with the N-(adamantyl-1)-2-aminoethanol of the structural formula III

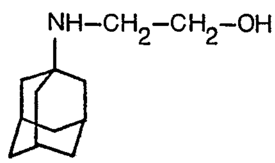

III

in a suitable inert solvent.

The salts of the compound I are obtained by the same procedure, noting that in order to finalize the reaction the resulting product is treated with the stoichiometric quantity of the acid in question (hydrochloric, sulphuric, tartaric, etc.)

Examples of the Chemical Preparation

Some examples of the method of preparation of the compounds of formula I are detailed below, all such details as are not essential to the invention being considered variables and not limititive thereto.

Example 1

Obtention of the p-chlorophenoxyacetate of N-(adamantyl-1)-2-aminoethanol, starting from a p-chlorophenoxyacetic acid halide.

In a 500 ml flask fitted with a mechanical stirrer, 22 g (0,11 mol) of N-(adamantyl-1)-2-aminoethanol dissolved in 250 ml of benzene are poured using a decanting funnel. 23 g (0.11 m) of p-chlorophenoxyacetyl chloride added in drops while stirring, the mixture then being stirred for 30 minutes. 120 ml of a 10% solution of sodium carbonate is then added and the resulting mixture is stirred for 10 minutes. The organic phase is decanted and the benzene removed by distillation. The residue is crystallized with petroleum ether. This yields 37 g (93%) of a white solid.

Elemental analysis;

| | | | | |
|---|---|---|---|---|
| Calculated: | C 66,01; | H 7,15; | Cl 9,77; | N 3,85; |
| Found: | C 66,2; | H 7,05; | Cl 9,68; | N 3,78; |

Example 2

Obtention of the p-chlorophenoxyacetate of N-(adamantyl-1)-2-aminoethanol starting from p-chlorophenoxyacetic acid.

In a 1 litre flask, provided with a Dean-Stark separator tube and reflux refrigerant, a mixture of 20,5 g (0,011 m) of p-chlorophenoxyacetic acid, 22 g (0,11 m) of N-(adamantyl-1)-2-aminoethanol, 98 g of conc. sulphuric acid and 700 ml of toluene is heated to boiling point during 24 hours. At the end of this period it is treated with an aqueous solution of 5% sodium carbonate to an alkali pH and washed with water; it is dried on anhydrous sodium sulphate and the toluene removed by distillation at reduced pressure. The crude so obtained is crystallized with petroleum ether. The yield is 35,2 g (88%) of a white solid.

Elemental analysis;

| | | | | |
|---|---|---|---|---|
| Calculated: | C 66,02; | H 7,15; | Cl 9,77; | N 3,85; |
| Found: | C 66,1; | H 7,10; | Cl 9,75; | N 3,80; |

Example 3

Obtention of the chlorhydrate of p-chlorophenoxyacetate of N-(adamantyl-1)-2-aminoethanol.

A solution of 60 g (0,16 m) of p-chlorophenoxyacetate of N-(adamantyl)-2-aminoethanol in 300 ml of ether is subjected to the passage of ClH gas until the precipitation of the solid product is complete. It is left to cool in a refrigerator during 6 hours and it is then filtered. The resulting solid is recrystallized with a mixture of ether and methanol. 61 g (92%) of the product are obtained.

Elemental analysis;

| | | | | |
|---|---|---|---|---|
| Calculated: | C 60,00; | H 6,75; | Cl 17,75; | N 3,50; |
| Found: | C 60,2; | H 6,70; | Cl 17,8; | N 3,55; |

In a similar manner and by using the stoichiometric quantities of the acid in question, the following salts are obtained.

| EXAMPLE | SALT | ELEMENTAL ANALYSIS<br>Calculated | <br>Found |
|---|---|---|---|
| 4 | Sulphate | C: 52,00 | C: 52,1 |
| | | H: 6,07 | H: 6,11 |
| | | Cl: 7,69 | Cl: 7,65 |
| | | N: 3,03 | N: 3,1 |
| | | S: 6,93 | S: 6,89 |
| 5 | Tartrate | C: 56,1 | C: 56,3 |
| | | H: 6,23 | H: 6,19 |
| | | Cl: 6,91 | Cl: 6,88 |
| | | N: 2,73 | N: 2,75 |
| 6 | Phosphate | C: 52,00 | C: 52,3 |
| | | H: 6,28 | H: 6,31 |
| | | Cl: 7,69 | Cl: 7,70 |
| | | N: 3,03 | N: 3,0 |
| | | P: 6,72 | P: 6,80 |

Evaluation of the pharmacological activity of the compound I

The following activities have been evaluated;

Nootropa activity

Tests were carried out on rats by the method of estimating the loss of memory induced by electric shock, loss of memory which was measured by the number of errors made in seeking the outlet from an acquatic labyrinth (Giurgea C.J. Pharmacol (Paris) *3*, 1, 17, *1972).

The compound I showed itself to be notably active, giving a 46,5% protection against the loss of memory, as is shown in the table 1, hereunder;

Activity on learning

This was studied by the test of learning in the acquatic labyrinth (Giurgea C.J. Pharmacol (Paris) *3*, 1, 17 (1972), the learning resulting in a decrease in the errors made and the time spent in reaching the exit. In another learning test (conditioning cage) the animals placed in the cage receive an electric shock which they can avoid by passing from one cell to another. Sara, S.J. and Col. Psychopharmacol (Ber.) 25, 32, (1972). The compound I has resulted in an increased learning facility as may be seen in the table 1.

Anti-depressant activity

This activity was evaluated by the method of examining the desperation behaviour in mice (Porsalt, T.D., Arch. Int Pharmacodyn 229, 327 (1977).

The state of despair was induced by placing the animal in a cylinder containing water and from which escape was impossible, then finding itself obliged to treadmill continually. After a period of vigorous activity the mouse takes up a characteristic immobile posture, this being capable of reduction by the use of various types of pharmaceutical anti-depressants. It was shown that the administration of the compound I resulted in moderate anti-depressant effects, as is shown in the table I.

Anti-catalepsy activity

This was the subject of Morpurgo tests (Arch. Int. Pharmacodyn, 137, 84 (1962), based on antagonizing the cataleptic condition in rats, induced by fluphenazine.

The compound I reduced slightly the cataleptic activity of the fluphenazine, as is shown in the table I.

TABLE I

Results of the pharmacological activity of Compound I

| TEST | PARAMETER | EVALUATION |
|---|---|---|
| Nootropa activity | Protection from memory loss due to electric shock. (number of errors) | 46,5% p<0,01 |
| | Decrease with respect to the control in the errors made in the aquatic labyrinth. (5th day). | 51,6% p<0,01 |
| Learning activity | Increase with respect to the control in the conditioning cage. (learning facility). | 14,4% p<0,001 |
| Anti-depressant activity. | Protection with respect to the control. | 45,9% 0<0,001 |
| Anti-cataleptic activity. | Protection against catalepsy by fluphenazine. | 25,6% p<0,01 |

Toxicity

From the Irwin or "screening" multi-dimensional test (Phsychopharmacologia (Berl), 13, 222—257 (1968), there was no evidence as to effects of non toxic doses. Toxic doses caused death accompanied by trembling and clonic convulsions.

The $LD_{50}$ of the compound I in the rat and the mouse is shown in the following table;

| ANIMAL | RAT | | MOUSE | |
|---|---|---|---|---|
| Route | i.p. | p.o. | i.p. | p.o. |
| $LD_{50}$ value mg/kg | 360 | >4000 | 415 | >4000 |

Therapeutical recommendations

Neuropsychic asthenia, problems of attention, memory defects, difficulty in learning, disorders in the capacity for mental concentration, mental involution in senility, amnesia, assistance in the treatment of Parkinson's disease, physical and mental debility, a propensity to a state of depression, irritability and lack of social adaptation due to depression, neuronal hypoxia, cerebral metabolism inadequacies, acute vascular complications, nervous sequelae to cerebral circulatory disorders, vascular toxic and traumatic comas, etc. Cerebral atherosclerosis.

Pharmaceutical presentation and dosage

*Tablets* of 50—500 mg of active principle, taken from 2 to 8 times a day.

*Syrops* of 10 to 200 mg/ml of active principle,.

The nature as well as the practical method of preparing the invention being adequately described, the procedures given above are capable of any modification in detail which does not alter the fundamental principle thereof or vary its essentiality.

**Claims**

1. A new compound derived from p-chlorophenoxy acetic acid, the p-chlorophenoxyacetate of N-(adamantyl-1)-2-aminoethanol, of the following structural formula I

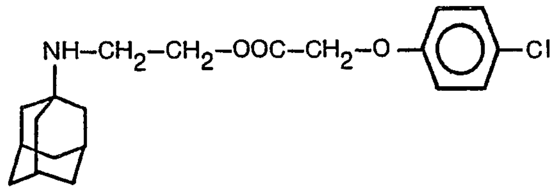

I

as well as its pharmaceutically acceptable salts.

2. The method of preparation of the new compound of general formula I, characterized in that, in accordance therewith an esterification reaction is carried out between the acylic compound of the following structural formula II:

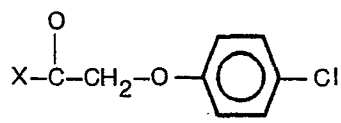

II

in which X can be an OH group or a halogen atom; (Cl or Br), and the N-(adamantyl-1)-2-aminoethanol of the following structural formula III

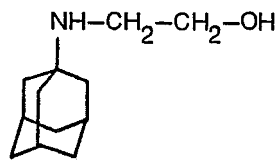

III

in a suitable inert solvent.

3. The method of preparation as in claim 2, characterized in that the salts of the compound of formula I are obtained starting from the said compound I, treating it with the stoichiometric quantity of the acid under consideration, such as hydrochloric, sulphuric, tartaric, or similar.

4. A pharmaceutical composition characterized by having as active ingredient the compound derived from the p-chlorophenoxyacetic acid of the structural formula I, as defined in claim 1.

5. A pharmaceutical composition characterized by having as active ingredient the compound derived from the p-chlorophenoxyacetic acid of the structural formula I, obtained by carrying out the procedure in accordance with either of the claims 2 or 3.

**Patentansprüche**

1. Von p-Chlorphenoxyessigsäure abgeleitetes p-Chlorphenoxyacetat des N-(Adamantyl-1)-2-aminoethanols der Strukturformel I

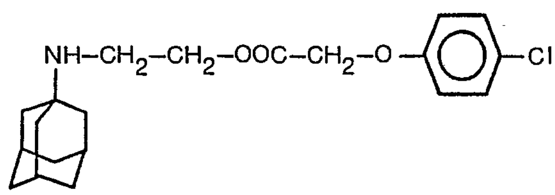

I

und dessen pharmazeutisch verträglichen Salze.

2. Verfahren zur Herstellung der Verbindung der allgemeinen Formel I, dadurch gekennzeichnet, daß eine Veresterungsreaktion zwischen der Acylverbindung der folgenden Strukturformel II

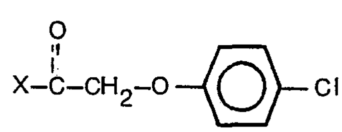

II

worin X eine OH-Gruppe oder ein Halogenatom (Cl oder Br) sein kann und dem N-(Adamantyl-1)-2-aminoethanol der folgenden Strukturformel III

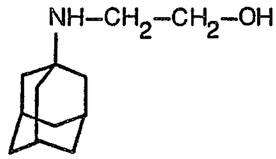

III

in einem geeigneten inerten Lösungsmittel durchgeführt wird.

3. Herstellungsverfahren nach Anspruch 2, dadurch gekennzeichnet, daß die Salze der Verbindung der Formel I ausgehend von der Formel I erhalten werden, indem die Verbindung I mit der stöchiometrischen Menge der in Frage kommenden Säure, wie Salzsäure, Schwefelsäure, Tartarsäure oder ähnliche Säuren behandelt wird.

4. Pharmazeutische Zusammensetzung, dadurch gekennzeichnet, daß sie als aktiven Bestandteil die von p-Chlorphenoxyessigsäure abgeleitete Verbindung der Strukturformel I gemäß der Definition in Anspruch 1 enthält.

5. Pharmazeutische Zusammensetzung, dadurch gekennzeichnet, daß sie als aktiven Bestandteil die von p-Chlorphenoxyessigsäure abgeleitete Verbindung der Strukturformel I enthält, die dadurch erhalten wird, daß das Verfahren gemäß Anspruch 2 oder Anspruch 3 durchgeführt wird.

**Revendications**

1. Nouveau composé dérivé de l'acide p-chlorophénoxyacétique, à savoir le p-chlorophénoxyacétate de N-(adamantyl-1)-2-aminoéthanol de formule de structure I

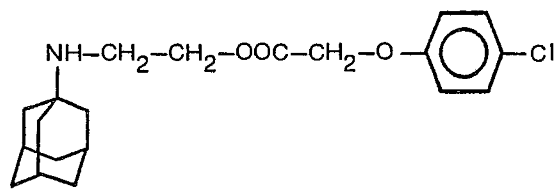

I

ainsi que ses sels pharmaceutiques acceptables.

2. Procédé de préparation du nouveau composé de formule générale I, caractérisé en ce que selon ce procédé, une réaction d'estérification est effectuée entre le composé acylé de formule de structure II

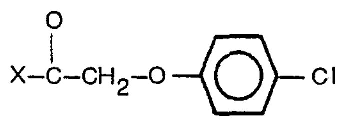

II

dans laquelle X peut être un groupe OH ou un atome d'halogène (Cl ou Br) et le N-(adamantyl-1)-2-aminoéthanol de formule de structure suivante III

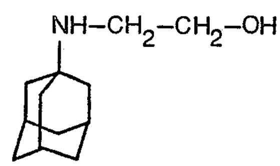

III

dans un solvant inerte approprié.

3. Procédé de préparation suivant la revendication 2, caractérisé en ce que les sels du composé de formule I sont obtenus au départ du composé I précité, en traitant celui-ci avec une quantité stoechiométrique de l'acide entrant en considération, tel que chlorhydrique, sulfurique, tartrique ou similaire.

4. Composition pharmaceutique caractérisée en ce qu'elle contient comme ingrédient actif le composé dérivé de l'acide p-chlorophénoxyacétique répondant à la formule de structure I, tel que défini à la revendication 1.

5. Composition pharmaceutique caractérisée en ce qu'elle contient comme ingrédient actif le composé dérivé de l'acide p-chlorophénoxyacétique répondant à la formule de structure I, obtenu en appliquant le procédé selon l'une quelconque des revendications 2 et 3.